(19) Europäisches Patentamt
European Patent Office
Office européen des brevets

(11) **EP 2 205 158 B1**

(12) **FASCICULE DE BREVET EUROPEEN**

(45) Date de publication et mention
de la délivrance du brevet:
**18.01.2012 Bulletin 2012/03**

(51) Int Cl.:
*A61B 6/02* *(2006.01)*    *G06T 5/50* *(2006.01)*
*G06T 11/00* *(2006.01)*    *G21K 1/10* *(2006.01)*

(21) Numéro de dépôt: **08847076.0**

(22) Date de dépôt: **04.11.2008**

(86) Numéro de dépôt international:
**PCT/EP2008/064963**

(87) Numéro de publication internationale:
**WO 2009/059982 (14.05.2009 Gazette 2009/20)**

(54) **PROCEDE ET DISPOSITIF D'IMAGERIE X OU INFRAROUGE A RAYONS RELECHIS PARALLELES PROCEDANT PAR SOUSTRACTION DES RAYONS DIRECTS**

VERFAHREN UND VORRICHTUNG ZUR RÖNTGEN- ODER INFRAROT-BILDGEBUNG MITHILFE PARALLEL REFLEKTIERTER STRAHLEN MIT ABZUG DER DIREKTEN STRAHLEN

METHOD AND DEVICE FOR X-RAY OR INFRARED IMAGING USING PARALLEL REFLECTED RAYS WITH SUBTRACTION OF THE DIRECT RAYS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **08.11.2007 FR 0707832**

(43) Date de publication de la demande:
**14.07.2010 Bulletin 2010/28**

(73) Titulaire: **Beyrard, Norbert**
**01220 Divonne-Les-Bains (FR)**

(72) Inventeur: **Beyrard, Norbert**
**01220 Divonne-Les-Bains (FR)**

(74) Mandataire: **Gaglione, Renaud**
**240, rue du Quart**
**01710 Thoiry (FR)**

(56) Documents cités:
**WO-A-2004/100790    WO-A-2006/107130**
**DE-A1- 10 139 384    DE-A1- 19 955 848**
**FR-A- 2 406 889    FR-A- 2 888 374**

**Description**

L'invention se rapporte à un procédé et à un dispositif d'imagerie X ou infrarouge, du type radiographie ou scannographie, et plus particulièrement à un procédé dans lequel, un corps à examiner étant reçu par un support,

-   on irradie ou illumine le corps à examiner à l'aide d'une source émettant un faisceau de rayons X ou lumineux, réfléchis en nappes parallèles par un réflecteur disposé entre la source et le corps à examiner,

-   on détecte une intensité atténuée en fonction d'une traversée des rayons X ou lumineux à travers le corps à examiner, à l'aide d'un détecteur irradié ou illuminé par le faisceau,

-   on convertit les intensités détectées en données pour déterminer une atténuation par le corps à examiner des rayons X ou lumineux, à l'aide d'un convertisseur analogique-numérique, et

-   à l'aide d'un ordinateur dûment programmé, on traite les données provenant de la conversion des intensités détectées pour aboutir à une image exprimant l'atténuation des rayons X ou lumineux par le corps examiné.

Dans le cas de la radiographie, on prend généralement deux vues du corps à examiner, l'une de face et l'autre de profil. Dans le cas de la scannographie, on est amené à prendre plusieurs vues sous différents angles de rotation pour aboutir à une image des coefficients d'atténuation du corps examiné dans des plans de coupe perpendiculairement à la rotation.

La source émet en général un faisceau divergent de telle sorte que les rayons qui frappent le détecteur s'inscrivent dans un cône centré autour d'une direction axiale de propagation. En fonction de l'angle solide du cône, les distances entre les points du corps examiné, représentés par les valeurs ponctuelles du coefficient d'atténuation, ne sont pas conservées mais subissent au contraire un effet de grossissement plus ou moins important. Dans le cas de la radiographie ou de la scannographie d'un individu, une telle déformation rend délicate l'exploitation de l'image par un chirurgien devant opérer l'individu. Une connaissance aussi parfaite que possible de l'anatomie d'un patient est encore plus souhaitable lorsque l'on cherche à détruire une singularité, par exemple un nodule cancéreux, au moyen d'un laser hautement énergétique émis en un temps très bref et transporté par une fibre optique ou focalisé sur la singularité. On consultera avec intérêt la demande internationale WO 2006/003311 déposée par le demandeur à ce sujet.

Le document DE-A-101 39 384 divulgue un procédé de radiographie qui remédie à la déformation des images obtenues des corps examinés, engendrée par l'utilisation de faisceaux divergents, en utilisant un réflecteur disposé entre la source et le corps à examiner pour réfléchir les rayons émis par la source suivant des nappes parallèles. Cependant, le réflecteur ne permet de réfléchir que les rayons émis par la source en dehors d'un angle solide limite s'appuyant sur le contour de sortie du réflecteur. A l'intérieur de cet angle solide limite, les rayons directs divergent sans jamais frapper le réflecteur. Pour les éliminer, il est prévu d'utiliser une plaque d'obturation disposée entre la source et corps à examiner, à l'entrée du réflecteur.

Le document WO 2004/100790 divulgue un dispositif d'imagerie à rayons X comprenant un ensemble de trois réflecteurs réfléchissant les rayons X émis par une source en rayons parallèles entre eux. Les réflecteurs s'étendent perpendiculairement à une direction axiale de propagation du faisceau, si bien que les rayons directs, c'est-à-dire non réfléchis par les réflecteurs, ne participent pas à l'irradiation du corps à examiner.

Le document WO 2006/107130 divulgue un dispositif d'imagerie à rayons X comprenant un filtre à rayons X quasi-monochromatique.

L'un des buts de l'invention est ainsi de modifier les procédés de radiographie ou de scannographie existants par rapport à l'élimination des rayons directs par une plaque d'obturation.

A cet effet, l'invention a pour objet un procédé conforme à celui rappelé en introduction, caractérisé en ce que :

-   on irradie ou illumine le corps à examiner une deuxième fois en l'absence du réflecteur, et
-   on effectue le traitement des données provenant des intensités détectées par le détecteur irradié ou illuminé en présence du réflecteur, diminuées des intensités détectées par le détecteur irradié ou illuminé en l'absence du réflecteur.

Le faisceau réfléchi en nappes parallèles de rayons X permet d'annuler l'effet de déformation du corps examiné, engendré par la divergence des rayons issus de la source. Les intensités détectées par le détecteur irradié ou illuminé en présence du réflecteur sont diminuées des intensités détectées par le détecteur irradié ou illuminé en l'absence du réflecteur pour soustraire la contribution des rayons directs, c'est-à-dire non réfléchis, et ne conserver par différence que la contribution des rayons réfléchis.

Dans le cas de la radiographie ou de la scannographie d'un patient, pour éviter de l'irradier deux fois, on préfèrera utiliser un fantôme, c'est-à-dire un mannequin en matière plastique reproduisant un corps humain en procédant de la façon suivante :

- on irradie ou illumine le fantôme en l'absence du réflecteur,

- on irradie ou illumine le fantôme en présence du réflecteur, et

- on irradie ou illumine le corps à examiner en présence du réflecteur.

Le fantôme permet d'établir un étalonnage du réflecteur donnant en chaque point du détecteur la proportion à retenir de l'intensité détectée propre aux rayons réfléchis. C'est la raison pour laquelle, dans le cas où l'on recherche une précision géométrique absolue, il sera préférable de procéder à deux irradiations du patient pour obtenir par différence, comme indiqué précédemment, la contribution des rayons réfléchis à la valeur totale de l'intensité détectée. Si un patient a été convenablement radiographié ou scannographié, on peut conserver les données acquises pour des examens ultérieurs, qui ne nécessiteront que l'irradiation en présence du détecteur. Autrement dit, les données antérieures remplaceront l'utilisation du fantôme dans les examens ultérieurs.

Le réflecteur permet de réfléchir en nappes parallèles, les rayons émis par la source en dehors d'un angle solide limite s'appuyant sur le contour de sortie du réflecteur. A l'intérieur de cet angle solide limite, les rayons directs ne viennent jamais frapper le réflecteur. La soustraction des rayons directs permet d'annuler par différence la contribution des rayons directs à l'image formée à partir des rayons réfléchis. Toutefois, dans une zone correspondant à la projection de la section d'entrée du réflecteur, les rayons directs atteignent seuls le détecteur et la soustraction des rayons directs conduit à l'annulation des intensités détectées et des données correspondantes.

Pour remédier à l'annulation des données, on réalise une image complémentaire après avoir déplacé l'un par rapport à l'autre, le corps à examiner et l'ensemble constitué par la source, le réflecteur et le détecteur. Cette opération sera facilitée par l'utilisation du fantôme ou par le rappel de données acquises dans une radiographie ou une scannographie antérieure.

L'invention s'étend à un dispositif spécialement conçu pour la mise en oeuvre du procédé de radiographie ou de scannographie qui vient d'être décrit, comprenant :

- un support pour recevoir un corps à examiner,

- une source émettant un faisceau de rayons X ou lumineux, réfléchis en nappes parallèles par un réflecteur disposé entre la source et le corps à examiner,

- un détecteur irradié ou illuminé par le faisceau, pour détecter une intensité atténuée en fonction d'une traversée des rayons X ou lumineux à travers le corps à examiner,

- un convertisseur analogique-numérique pour convertir les intensités détectées en données pour déterminer une atténuation par le corps à examiner des rayons X ou lumineux, et

- un ordinateur dûment programmé pour traiter les données provenant de la conversion des intensités détectées pour aboutir à une image exprimant l'atténuation des rayons X ou lumineux par le corps examiné,

caractérisé en ce que :

- le réflecteur est monté amovible par rapport à la source, pour irradier ou illuminer le corps à examiner en l'absence et en présence du réflecteur,

et en ce que :

- l'ordinateur est dûment programmé pour effectuer le traitement des données provenant des intensités détectées par le détecteur irradié ou illuminé en présence du réflecteur, diminuées des intensités détectées par le détecteur irradié ou illuminé en l'absence du réflecteur.

Dans un premier mode préféré d'exécution de l'invention, le réflecteur est un paraboloïde dont le foyer est confondu avec le foyer de la source, pour paralléliser les rayons réfléchis en un faisceau cylindrique. Toutefois, fabriquer par usinage un tel paraboloïde avec une haute précision oblige à une réalisation mécanique coûteuse. On utilisera de préférence un réflecteur tronconique selon les deuxième, troisième ou quatrième modes d'exécution indiqués ci-dessous.

Dans ce deuxième mode d'exécution, le réflecteur comprend une succession de troncs de cône étagés, dont la droite génératrice est la plus proche possible de l'arc de la parabole de référence pour minimiser les écarts à chaque étage. Cet agencement est plus simple à fabriquer, mais une fraction de l'énergie émise par la source est perdue, dans la mesure où la section de sortie du réflecteur, circulaire, ne coïncide plus avec l'aire rectangulaire ou carrée de détection du détecteur.

Dans le troisième mode d'exécution de l'invention, le réflecteur comprend une succession de troncs de pyramide étagés de section carrée, hexagonale ou octogonale, choisis pour minimiser les écarts, à chaque étage, avec une parabole de référence. Cet agencement permet avantageusement de mieux faire, voire exactement, coïncider la section de sortie du réflecteur avec l'aire de détection, rectangulaire ou carrée, du détecteur, de sorte à optimiser le rendement d'irradiation ou d'illumination.

Dans le quatrième mode d'exécution de l'invention, le réflecteur comprend une succession de troncs de cône étagés, revêtus d'une épaisseur de métal lourd, par exemple le plomb, variant suivant arc d'une parabole de référence.

Avantageusement, les troncs de cône ou les troncs de pyramide s'ouvrent en deux demi-troncs rétractables pour permettre leur translation par rapport à la source.

L'invention s'étend à un programme d'ordinateur pour la mise en oeuvre d'un tel procédé lorsqu'il est chargé dans un ordinateur.

D'autres avantages de l'invention apparaîtront à la lumière de la description des modes de réalisation illustrés par les dessins.

La figure 1 est une vue d'ensemble d'un dispositif à radiographie ou à scannographie selon l'invention.

La figure 2 illustre la géométrie angulaire de la réflexion ou de la réverbération d'un rayon incident sur une plaque.

La figure 3 est un tableau donnant la valeur de l'angle d'incidence d'un rayon au point (x,y) de la parabole d'équation $y^2 = 2 * 1 * x$.

La figure 4 est une vue en perspective d'un réflecteur comprenant trois étages, chacun représenté par un tronc de pyramide de section carrée.

La figure 5 est une vue en perspective d'un réflecteur comprenant trois étages, chacun représenté par un tronc de pyramide de section hexagonale.

La figure 6 est une vue en perspective d'un réflecteur comprenant trois étages, chacun représenté par un tronc de cône.

La figure 7 est un tableau donnant les valeurs de calcul de l'étagement de quatre troncs de cône pour former un réflecteur.

La figure 8 montre en coupe un réflecteur 15 comprenant quatre troncs de cône étagés, choisis pour minimiser à chaque étage un écart par rapport à un arc d'une parabole de référence.

La figure 9 montre en coupe un réflecteur comprenant trois troncs de cône chacun revêtus intérieurement d'une épaisseur de métal lourd variant suivant un arc d'une parabole de référence.

La figure 10 est une représentation schématique en coupe d'un caisson à vide utilisé pour la fabrication du tronc de cône illustré par la figure 9.

La figure 11 est une représentation schématique des rayons émis par une source et reçus par un détecteur en présence d'un réflecteur dans un dispositif selon l'invention, pour une première position du corps à examiner.

La figure 12 est une représentation schématique des rayons émis par une source et reçus par un détecteur en présence d'un réflecteur dans un dispositif selon l'invention, pour une deuxième position du corps à examiner.

La figure 13 est une représentation schématique de l'aire de détection utilisée pour la formation de la première ou de la deuxième image selon la figure 11 ou 12, tronquée par deux plans P1 et P2.

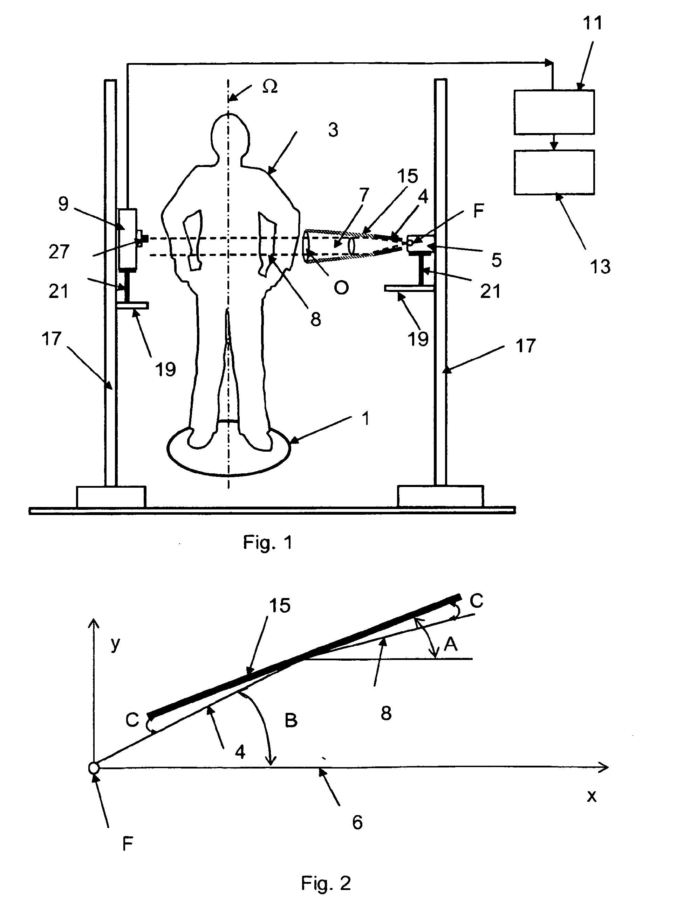

Un dispositif spécialement conçu pour la radiographie ou la scannographie comprend, figure 1 :

- un support 1 pour recevoir un corps à examiner 3,

- une source 5 émettant un faisceau 7 de rayons X ou lumineux pour irradier ou illuminer le corps à examiner 3,

- un détecteur 9 irradié ou illuminé par le faisceau 7, pour détecter une intensité atténuée en fonction d'une traversée des rayons X ou lumineux à travers le corps à examiner 3,

- un convertisseur 11 analogique-numérique pour convertir les intensités détectées en données pour déterminer une atténuation par le corps à examiner 3 des rayons X ou lumineux, et

- un ordinateur 13 dûment programmé pour traiter les données provenant de la conversion des intensités détectées pour aboutir à une image exprimant l'atténuation des rayons X ou lumineux par le corps examiné 3.

La source 5, comprenant un tube à rayons X, et le détecteur 9 sont déplacés le long d'une potence verticale 17 à l'aide d'un premier vérin 19 à grande course de déplacement. Un deuxième vérin 21 à faible course de déplacement est prévu pour déplacer la source 5 et le détecteur 9 selon une course d'ajustement.

Le dispositif comprend un réflecteur 15 disposé entre la source 5 et le corps à examiner 3, pour irradier ou illuminer le corps à examiner 3 en réfléchissant les rayons émis par la source 5 suivant des nappes parallèles. L'ordinateur 13 est dûment programmé pour effectuer le traitement des données provenant des intensités détectées par le détecteur 9 irradié ou illuminé en présence du réflecteur, diminuées des intensités détectées par le détecteur 9 irradié ou illuminé en l'absence du réflecteur 15.

Le réflecteur 15 est disposé en regard du foyer F de la source 5. Il est monté amovible par rapport elle pour être retiré lorsque l'on doit détecter les intensités en l'absence du réflecteur. Lorsque l'on doit détecter les intensités en présence du réflecteur, ce dernier est déplacé avec la source par le premier vérin 19 et par le deuxième vérin 21.

En incidence rasante, les rayons X émis par la source 5 se réfléchissent totalement ou partiellement sur les parois du réflecteur 15. On détermine l'angle θ en dessous duquel la réflexion est totale par la formule suivante :

$$\theta < \lambda / (5{*}10^{-8})$$

Les longueurs d'onde λ sont liées à la tension d'alimentation E par la formule suivante:

$$E\ (kV) = 12.38 / \lambda\ (Ang)$$

Le calcul conduit rapidement aux résultats suivants :

- pour 125 kV θ = 1,12 °

- pour 70 kV     θ = 2 °

- pour 35 kV     θ = 4 °

Suivant un premier mode d'exécution de l'invention, le réflecteur 15 est un paraboloïde de révolution pour paralléliser les rayons réfléchis en un faisceau cylindrique.

Rappelons qu'une parabole est définie par une fonction :

$$y^2 = 2 * \rho * x$$

Le paramètre ρ détermine la section de sortie du paraboloïde de révolution. A titre d'exemple, lorsque le paramètre ρ est égal à l'unité, la section de sortie à 50 cm du foyer du paraboloïde est un cercle de rayon égal à 10 cm. On peut ainsi irradier ou illuminer le corps à examiner 3 sur un cercle de 20 cm de diamètre centré sur la projection du foyer F de la source 5 sur le détecteur 9.

Si le foyer de du tube à rayons X se confond avec le foyer de la parabole générant le paraboloïde, le faisceau sera totalement réfléchi ou partiellement réverbéré, de sorte à ce que les rayons réfléchis ou réverbérés qui touchent et traversent l'objet à examiner 3 soient horizontaux, lorsque l'axe du tube à rayons X est lui aussi horizontal.

Les conditions de la réflexion ou de la réverbération dépendent de l'angle d'un rayon incident avec la tangente à la parabole au point d'incidence. Le graphique de la figure 2 illustre la géométrie angulaire de la réflexion ou de la réverbération d'un rayon incident sur une plaque 15. L'angle incident C sur la plaque est égal à la différence entre l'angle B du rayon incident 4 avec l'horizontale, représentée par la direction 6 de l'axe du tube à rayons X, et l'angle A de la plaque avec l'horizontale 6. Lorsque la plaque 15 est tangente au point d'incidence à un paraboloïde dont le foyer se confond avec le foyer de la source 5, le rayon réfléchi 8 est horizontal.

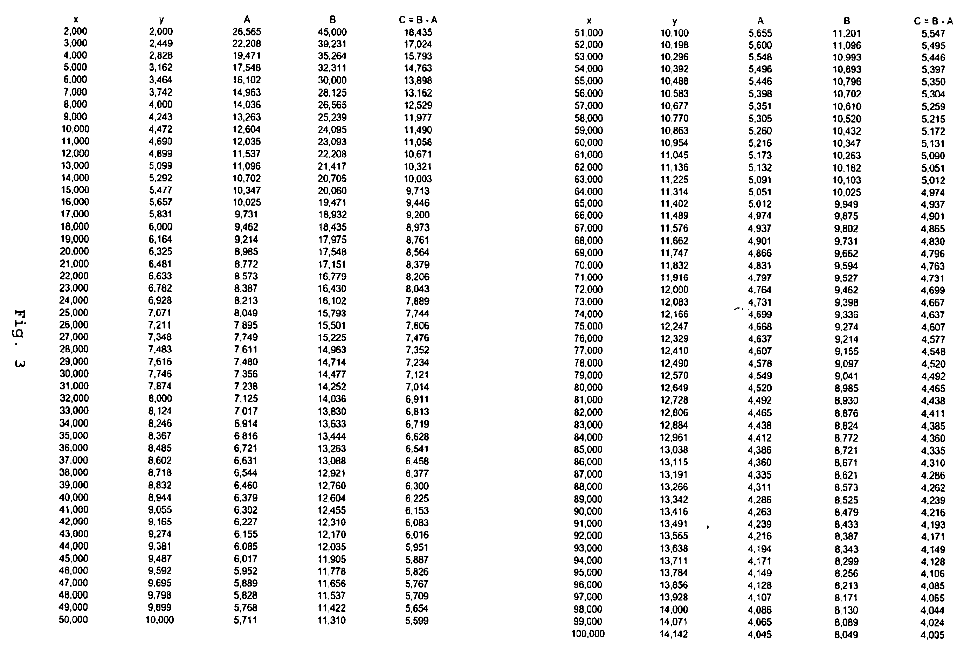

Le tableau de la figure 3 donne la valeur de l'angle d'incidence au point (x,y) de la parabole d'équation $y^2 = 2 * 1 * x$, en fonction de l'angle B du rayon incident 4 avec l'horizontale et de l'angle A de la tangente au point. On constate que l'écart entre l'angle incident C = B - A et la pente A de la parabole au point (x,y) devient rapidement négligeable au fur et à mesure que l'on s'éloigne du foyer F. Un écart nul réalise la condition parfaite pour réfléchir le rayon incident 4 un rayon horizontal 8. On constate également que l'angle d'incidence C décroît par valeur supérieure vers la valeur égale à 4° à une distance de 100 cm du foyer.

Pour $\rho$ = 1 et E = 75000 volts, l'angle de réflexion sur la surface du réflecteur, calculée à partir de la tangente au point considéré, entraîne une réflexion totale pour des valeurs angulaires inférieures à 2°. Le taux de réverbération mesure le rapport entre la réflexion partielle et la réflexion totale théorique. Ce taux est généralement acceptable pour des angles inférieurs à 10°.

Dans un mode d'exécution d'un dispositif selon l'invention, le réflecteur 15 comprend une succession de troncs de pyramide étagés de section carrée, hexagonale ou octogonale, choisis pour minimiser à chaque étage l'écart avec un arc d'une parabole de référence.

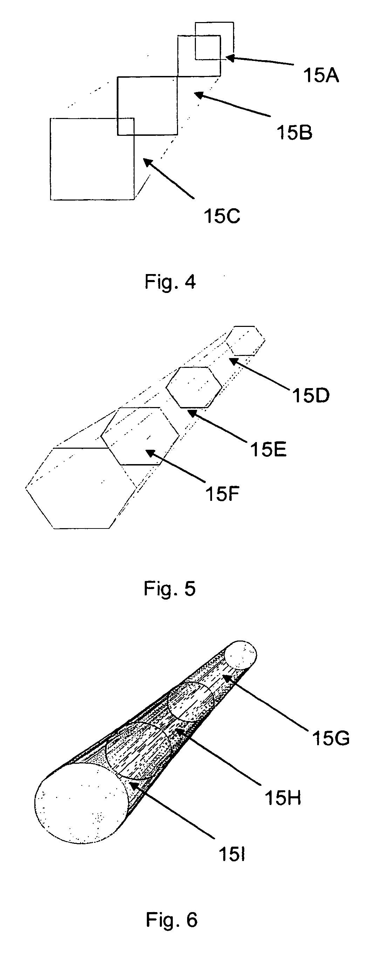

Figure 4, un réflecteur de ce type comprend trois étages, chacun représenté par un tronc de pyramide 15A, 15B et 15C. La construction du réflecteur est telle que la base carrée du deuxième tronc de pyramide 15B coïncide avec le sommet carré du premier tronc de pyramide 15A. De même, la base carrée du troisième tronc de pyramide 15C coïncide avec le sommet carré du deuxième tronc de pyramide 15B. Les troncs de pyramide de section carrée sont calculés pour être tangents à un paraboloïde de révolution. De préférence, la section est choisie, à chaque étage, à mi-chemin entre le polygone inscrit dans le cercle de la section homologue du paraboloïde de référence et le polygone tangent à cette section homologue.

Figure 5, le réflecteur comprend trois étages, chacun représenté par un tronc de pyramide à section hexagonale 15D, 15E et 15F. La construction de ce réflecteur est la même que celle du réflecteur décrit précédemment.

Ces réflecteurs à troncs de pyramide étagés ne comportent que des éléments de plaque aisément assemblés entre eux. Cependant, ils possèdent une symétrie d'ordre inférieur, 4 pour la base carrée, 6 pour la base hexagonale, à la symétrie de révolution du paraboloïde décrit dans le premier mode d'exécution. De ce fait, les rayons réfléchis ne sont pas tous parallèles et le faisceau réfléchi n'est pas parfaitement cylindrique.

Dans autre mode d'exécution d'un dispositif selon l'invention, le réflecteur 15 comprend une succession de cônes étagés, coaxiaux et choisis là encore pour minimiser à chaque étage l'écart par rapport à un arc d'une parabole de référence.

Figure 6, un réflecteur de ce type comprend trois étages, chacun représenté par un tronc de cône 15G, 15H et 15I. Là encore, la construction du réflecteur est telle que la base circulaire du deuxième tronc de cône 15H coïncide avec le sommet circulaire du premier tronc de cône 15G. De même, la base circulaire du troisième tronc de cône 15I coïncide avec le sommet circulaire du deuxième tronc de pyramide 15H.

La droite génératrice de chaque tronc de cône est calculée pour être en tous points aussi proche que possible de l'arc correspondant de la parabole génératrice d'un paraboloïde de référence. Deux étagements sont prévus, l'un dans lequel les cônes ont tous la même hauteur suivant la direction axiale et l'autre dans lequel ils ont chacun une hauteur axiale déterminée par calcul pour correspondre à un rapport constant de décroissance du rayon de courbure du paraboloïde de révolution.

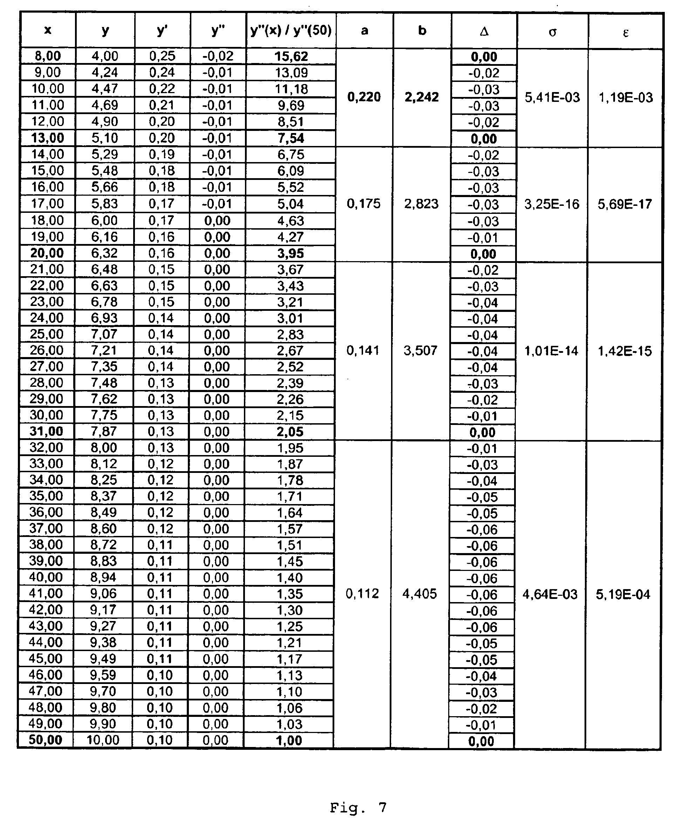

Le tableau de la figure 7 illustre les calculs effectués pour un rapport de décroissance égal à 2. Autrement dit, l'étagement choisi est celui pour lequel le rayon de courbure du paraboloïde décroît de moitié sur la hauteur de chaque cône étagé. Dans le tableau de la figure 7,

- la colonne x indique les abscisses de l'axe central du paraboloïde,

- la colonne y indique les ordonnées du paraboloïde,

- la colonne y' indique la dérivée première de la fonction y,

- la colonne y" indique la dérivée seconde de la fonction y, soit encore la valeur ponctuelle du rayon de courbure,

- la colonne y"(x) / y"(50) indique le coefficient relatif de courbure rapporté au rayon de courbure à la section de sortie du réflecteur paraboloïde,

- la colonne a indique la pente de la droite génératrice d'un tronc de cône,

- la colonne b indique l'ordonnée à l'origine de la droite génératrice d'un tronc de cône,

- la colonne Δ indique l'écart entre la droite d'équation y = a x + b, génératrice d'un tronc de cône, et le paraboloïde, pour quatre troncs de cône définis par quatre hauteurs axiales,

- la colonne σ indique l'écart type de chacune des quatre distributions associées aux quatre troncs de cône, et

- la colonne ε indique le coefficient d'erreur pour chaque distribution, c'est-à-dire le rapport entre l'écart-type et la valeur moyenne de la distribution.

Avec une section de sortie O du réflecteur disposée à 50 cm du foyer du paraboloïde, confondu avec le foyer F du tube à rayons X, les quatre troncs de cône s'étendent, pour le premier, entre 8 cm et 13 cm, pour le deuxième, entre 13 cm et 20 cm, pour le troisième, entre 20 cm et 31 cm, et pour le quatrième, entre 31 cm et 50 cm. Pour les quatre distributions, les écarts-types sont très faibles et les coefficients d'erreur au plus égaux à 1 pour 1000, voire infinitésimaux. Etant donné les angles de divergence des faisceaux X usuels, les phénomènes de réflexion concernent principalement les étages supérieurs 20 - 31 et 31 - 50, où l'erreur est infinitésimale.

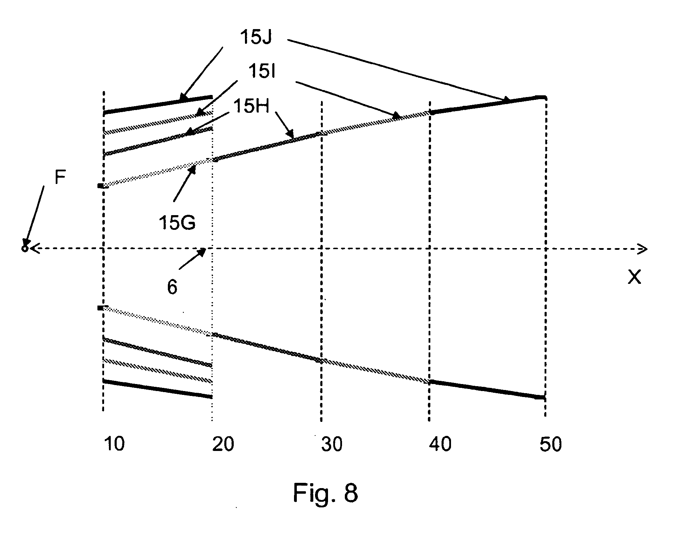

Un étagement de quatre troncs de cône de hauteur axiale constante, par exemple égale à 10 cm, conduit également à de bons résultats. La figure 8 montre un réflecteur 15 comprenant quatre troncs de cône 15G, 15H, 15I et 15J étagés de 10 cm en 10 cm, la section de sortie du réflecteur étant disposée à 50 cm du foyer F du tube à rayons X, confondu avec le foyer du paraboloïde de révolution auquel les troncs de cône sont tangents.

Les troncs de cône sont fabriqués à partir de découpe dans un plan d'une surface limitée par deux arcs de cercle concentriques tels que la distance entre les deux arcs mesurée le long d'un rayon soit toujours la même. La découpe est effectuée dans un ensemble feuilleté puis plié sur un cône de référence pour souder les deux bords opposés. L'ensemble feuilleté comprend :

- une plaque en acier inoxydable, de 1 mm d'épaisseur,

- une plaque en plomb, de 2 à 3 mm d'épaisseur, et

- une deuxième plaque en acier inoxydable, de 1 mm d'épaisseur.

L'ensemble feuilleté est comprimé pour que le plomb et l'acier soient parfaitement collés. On prévoit d'ajouter un revêtement en métal lourd, tel l'or ou le platine, pour obtenir une surface parfaitement régulière sur la face intérieure du réflecteur en contact avec le faisceau.

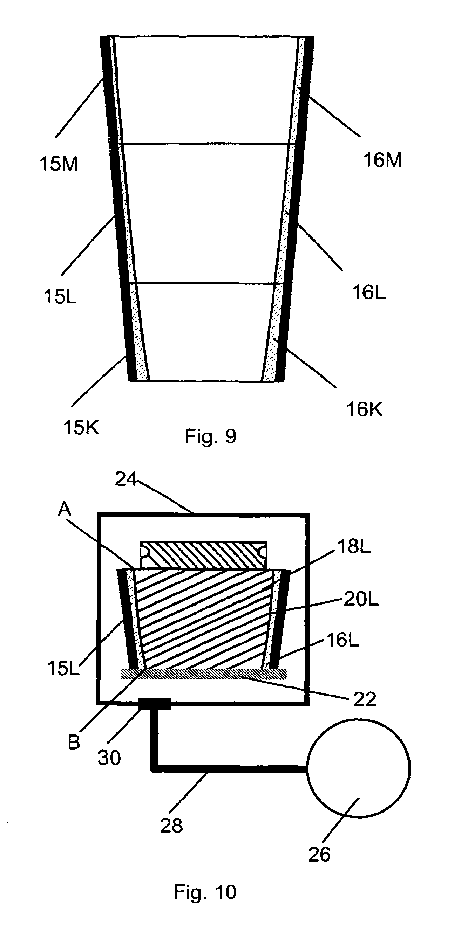

Dans un quatrième mode de réalisation du dispositif selon l'invention, figure 9, le réflecteur comprend une succession de troncs de cône étagés 15K, 15L et 15M, revêtus sur leur paroi intérieure d'une épaisseur, respectivement 16K, 16L et 16M, d'un métal lourd, par exemple le plomb. Les troncs de cône sont fabriqués comme indiqué précédemment, par découpe d'une plaque, par exemple en acier inoxydable.

On fabrique parallèlement une forme pleine, par exemple en aluminium, ayant un profil extérieur épousant un arc d'une parabole de référence. Figure 10, la forme pleine 18L présente une paroi extérieure 20L dont le profil longitudinal épouse l'arc A-B d'une parabole de référence. La forme pleine 18L est disposée à l'intérieur du tronc de cône 15L en reposant sur un support 22. Une feuille de plomb est insérée entre la paroi extérieure 20L et la paroi intérieure du tronc de cône 15L et l'ensemble est disposé dans un caisson à vide 24 raccordé à une pompe à vide 26, par exemple à palettes lubrifiées, par l'intermédiaire d'un tube d'aspiration 28 et d'une vanne 30. Le caisson à vide comprend une ouverture fermée de façon étanche par un couvercle.

On crée dans le caisson 24 un vide pouvant atteindre quelques millibars en actionnant la pompe à palettes lubrifiées 26 puis on ferme la vanne 30 et on retire le raccord d'aspiration 28. Le caisson 24 est ensuite disposé dans un four dont la température est réglée pour atteindre le point de fusion de la feuille de métal lourd insérée entre le tronc de cône 15L et la forme pleine 18L, par exemple 327 °C dans le cas du plomb. La montée en température et le refroidissement sont réglés pour éviter la formation de contraintes dans l'épaisseur de plomb 16L, dues à la différence des coefficients de dilatation thermique du tronc de cône 15L et de la forme pleine 18L. A l'issue du refroidissement, on casse le vide dans le caisson 24 puis on retire la forme pleine 18L. Le démoulage est facilité si l'on a préalablement enduit la forme pleine 18L de talc.

Les troncs de cône sont avantageusement montés rétractables les uns par rapport aux autres, pour permettre de passer aisément d'une irradiation en présence du réflecteur à une irradiation en l'absence du réflecteur. En référence à la figure 8, il est prévu de monter inamovible le premier cône 15G, le plus près de la source 5, et d'ouvrir les autres cônes 15H, 15I et 15J en deux demi-cônes pour les rétracter en translation parallèlement à la direction horizontale 6 de l'axe du tube à rayons X, afin de faciliter le repliement et le dépliement du réflecteur 15 par rapport à la source 5.

Le dispositif qui vient d'être décrit est mis en oeuvre par un procédé du type radiographie ou scannographie, dans lequel, le corps à examiner 3 étant reçu par le support 1,

- on irradie ou illumine le corps à examiner 3 à l'aide de la source 5 émettant un faisceau 7 de rayons X ou lumineux, réfléchis en nappes parallèles par un réflecteur disposé entre la source 5 et le corps à examiner 3,

- on détecte une intensité atténuée en fonction d'une traversée des rayons X ou lumineux à travers le corps à examiner 3, à l'aide du détecteur 9 irradié ou illuminé par le faisceau,

- on convertit les intensités détectées en données pour déterminer une atténuation par le corps à examiner des rayons X ou lumineux, à l'aide du convertisseur 11 analogique-numérique, et

- à l'aide de l'ordinateur 13 dûment programmé, on traite les données provenant de la conversion des intensités détectées pour aboutir à une image exprimant l'atténuation des rayons X ou lumineux par le corps examiné 3, et dans lequel selon l'invention,

- on irradie ou illumine le corps à examiner une deuxième fois en l'absence du réflecteur, et

- on effectue le traitement des données provenant des intensités détectées par le détecteur 9 irradié ou illuminé en présence du réflecteur, diminuées des intensités détectées par le détecteur 9 irradié ou illuminé en l'absence du réflecteur 15.

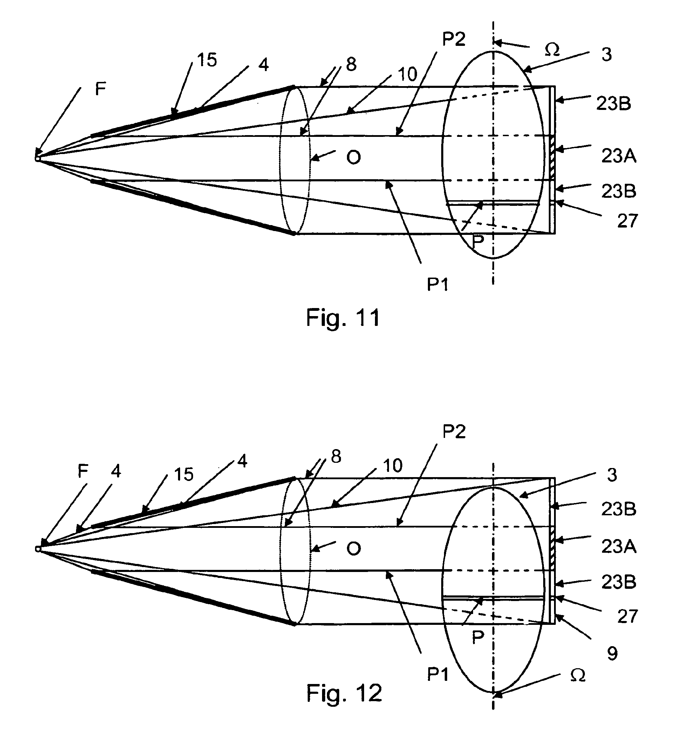

Comme indiqué précédemment, le réflecteur 15 permet de rendre horizontaux les rayons émis par la source 5 en dehors d'un angle solide limite, à l'intérieur duquel les rayons directs ne viennent jamais frapper le réflecteur 15. Cet angle solide s'appuie sur la section de sortie O du réflecteur 15. La figure 11 en donne une illustration par la représentation schématique des rayons émis par la source 5 et reçus par le détecteur 9 en présence d'un réflecteur 15 paraboloïde de révolution. On distingue entre trois zones de l'aire de détection 23 du détecteur 9, du centre vers la périphérie:

- une première zone 23A, circulaire, correspondant à la projection de la section d'entrée E du réflecteur sur l'aire de détection 23 du détecteur 9, irradiée ou illuminée uniquement par des rayons directs 10, c'est-à-dire des rayons non réfléchis,

- une deuxième zone 23B, annulaire, irradiée ou illuminée à la fois par des rayons directs 10 et par des rayons réfléchis 8, et

- une troisième zone non illustrée, annulaire, irradiée ou illuminée uniquement par des rayons directs.

Pour soustraire les rayons directs 10 et ne conserver que les rayons réfléchis 8 dans la deuxième zone 23B, on procède à deux irradiations ou illuminations, l'une en présence du réflecteur 15 et l'autre en l'absence de ce dernier. Dans le cas d'une radiographie ou d'une scannographie humaine, on préfère utiliser un fantôme comme indiqué précédemment, ce qui évite d'irradier ou d'illuminer deux fois le patient.

Par l'effet de la soustraction des rayons directs 10, les données correspondant à la première zone 23A sont nulles. Aussi, pour obtenir une image complète sur toute l'étendue de l'aire de détection du détecteur, il est prévu de procéder en deux étapes :

- dans une première étape, figure 11, on procède à une irradiation ou illumination du corps à examiner 3 dans une première position de ce dernier vis-à-vis de l'ensemble constitué par la source 5, le réflecteur 15 s'il est présent et le détecteur 9, pour former une première image correspondant à la zone 23B de l'aire de détection du détecteur 9, et

- dans une deuxième étape, figure 12, on déplace l'ensemble précédent 5, 15, 9 par rapport au corps à examiner 3 dans une deuxième position pour former une image complémentaire, correspondant à la zone 23B de l'aire de détection du détecteur 9 dans cette deuxième position et englobant la zone 23A de ce même détecteur 9 lorsqu'il se trouvait dans la première position.

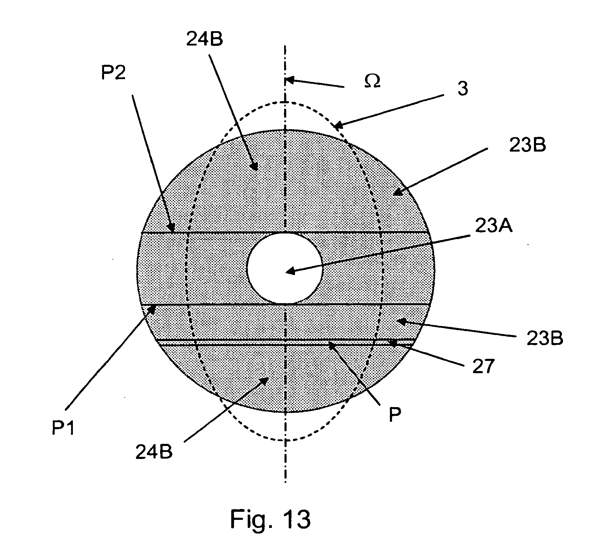

Comme illustré par la figure 13, seule une région 24B de la zone annulaire 23B de l'aire de détection, tronquée par deux plans P1 et P2 parallèles aux nappes de rayons réfléchis et tangents à la zone circulaire 23A de l'aire de détection, est utilisée pour la formation de la première et de la deuxième image.

L'ordinateur 13 est dûment programmé pour traiter les données acquises aux première et deuxième étapes pour aboutir à une seule image, exprimant l'atténuation des rayons X ou lumineux par le corps examiné et correspondant aux zones 23A et 23B de l'aide de détection du détecteur 9.

Le réflecteur 15 réfléchissant les rayons émis par la source 5 suivant des nappes parallèles, et de préférence suivant un faisceau cylindrique, chaque rayon réfléchi parcourt une distance qui lui est propre entre la source 5 et le point d'incidence sur le réflecteur 15 d'une part, et entre ce point et le détecteur 9 d'autre part. Les différences de parcours sont calculables, plus aisément lorsque le réflecteur est un paraboloïde de révolution ou une succession étagée de troncs de cône, que lorsqu'il comprend une succession étagée de troncs de pyramide à base carrée ou hexagonale.

Pour tenir compte de ces différences de parcours entre les rayons réfléchis, mais également de différences de réverbération aux différents points d'incidence du réflecteur 15, il est prévu de procéder à un étalonnage du réflecteur 15, par le calcul ou de façon expérimentale. On prévoit encore de procéder à un contrôle de l'étalonnage du réflecteur dans le temps, compte tenu d'une dégradation possible de l'état de la surface réfléchissante.

Dans la suite de l'exposé de l'invention, on se concentrera sur un procédé d'imagerie X ou infrarouge du type scannographie ainsi que sur un dispositif spécialement conçu pour la mise ne oeuvre d'un tel procédé. Pour un rappel du principe à la base de la scannographie ainsi que pour un exposé complet du procédé de scannographie par génération directe auquel il est fait référence désormais, on consultera avec intérêt la demande française FR 2 888 374 et la demande internationale WO 2007/006560 déposées par le demandeur. On pourra également consulter la demande française FR 2 871 911 et la demande internationale WO 2006/003312 déposées par le demandeur pour un procédé de scannographie par amplification matricielle et ajustement point par point.

Le procédé de scannographie est plus particulièrement défini par le fait qu'au cours de l'acquisition des données :

- on fait tourner d'un angle de rotation le support 1 monté mobile autour d'un axe de rotation $\Omega$ par rapport à la source 5 et au détecteur 9 montés sur les potences 17 servant de bâti ou tourner d'un angle de rotation la source et le détecteur montés sur un bâti mobile autour de l'axe de rotation par rapport au support,

- on convertit les intensités détectées en données pour déterminer une atténuation par le corps à examiner 3 des rayons X ou lumineux dans un plan de coupe perpendiculaire à l'axe de rotation $\Omega$, à l'aide du convertisseur 11 analogique-numérique,

et par le fait qu'au cours du traitement des données, à l'aide de l'ordinateur 13 dûment programmé, on effectue les étapes suivantes :

(1) construire un vecteur générateur colonne et un vecteur générateur ligne dont les termes sont respectivement constitués par les données $(c_i)$ et $(\rho_j)$ provenant de la conversion des intensités détectées dans une bande 27 du détecteur 9 pour respectivement un premier et un deuxième angle de rotation, de préférence différents entre eux de 90 degrés, du support 1 ou du bâti mobile autour de l'axe de rotation, les données étant moyennées respectivement en n et m valeurs moyennes correspondant à un quadrillage en n x m zones élémentaires du plan de coupe P du corps à examiner 3, perpendiculaire à l'axe de rotation $\Omega$ et contenant la bande 27 du détecteur 9,

(2) construire une matrice initiale (n,m) avec les termes des deux vecteurs générateurs, en affectant à chaque zone élémentaire un terme de ligne et de colonne (Bij) représentant un coefficient d'atténuation et défini par la demi-somme, du terme homologue $(c_i)$ du vecteur générateur colonne divisé par le nombre (m) de termes du vecteur générateur ligne et du terme homologue $(\rho_j)$ du vecteur générateur ligne divisé par le nombre (n) de termes du vecteur générateur colonne,

$$B_{ij} = \frac{1}{2}\left(\frac{\rho_j}{n} + \frac{c_i}{m}\right)$$

(3) ajuster le coefficient d'atténuation en chaque zone élémentaire en utilisant la formule suivante :

$$C_{ij} = \frac{\rho_j}{n} + \frac{c_i}{m} - \frac{1}{2nm}\left(\sum_{i=1}^{n} c_i + \sum_{j=1}^{m} \rho_j\right)$$

où, dans cette formule,

$C_{ij}$ = la valeur recherchée du coefficient d'atténuation de la zone élémentaire (i,j) du quadrillage
$B_{ij}$ = la valeur estimée initialement
(n) = le nombre de lignes de la matrice initiale
(m) = le nombre de colonnes de la matrice initiale
$\rho_j$ est le j-ème terme du vecteur générateur de ligne
$c_i$ est le i-ème terme du vecteur générateur de colonne

pour aboutir à une image du plan de coupe du corps examiné sous les premier et deuxième angles de rotation, correspondant à une matrice ajustée pour laquelle les valeurs de bordure de ligne et de colonne calculées à l'aide des valeurs ajustées ($C_{ij}$) sont égales, pour chaque ligne et pour chaque colonne, respectivement aux termes des vecteurs générateurs ligne et colonne,

$$\sum_{j=1}^{m} C_{ij} = c_i$$

$$\sum_{i=1}^{n} C_{ij} = \rho_j$$

(4) répéter les étapes (1) à (3) pour des données acquises avec différentes paires d'angles de rotation pour aboutir respectivement à différentes matrices ajustées correspondant à différentes images du plan de coupe du corps examiné sous les différentes paries d'angles de rotation, et

(5) à l'aide d'un opérateur de rotation, superposer sur une même paire d'angles toutes les matrices ajustées, et

(6) afficher à l'écran de l'ordinateur une image de synthèse du plan de coupe du corps examiné correspondant à une matrice de synthèse des coefficients d'atténuation en chaque zone élémentaire (i,j) du quadrillage, obtenus par une moyenne terme à terme de toutes les matrices ajustées et superposées.

Pour obtenir une image comportant (m x n) points du corps à examiner 3 dans le plan de coupe P, on construit, aux étapes (1) et (2), une matrice initiale de (m x n) termes par une estimation de chacun des termes des vecteurs générateurs colonne et ligne à partir des données obtenues à la suite seulement de deux irradiations du corps à examiner sous deux angles de rotation, par exemple décalés de 90 degrés.

A l'étape (3), on procède à un *ajustement* des termes estimés de la matrice initiale par rapport aux n et m valeurs moyennes d'intensité détectées en tenant compte des valeurs de bordure. On aboutit ainsi à une première matrice ajustée correspondant à une première image ajustée. La méthode du calcul d'ajustement est incorporée intégralement par référence à la demande internationale WO 2007/006560.

En pratique, les étapes (2) et (3) sont regroupées en une seule étape dite de génération directe des valeurs recherchées du coefficient d'atténuation $C_{ij}$ de l'image du plan de coupe du corps examiné sous les premier et deuxième angles de rotation. La formule utilisée à l'étape (3) ne nécessite en effet que la connaissance des données $c_i$ et $\rho_j$ provenant des intensités détectées dans la bande du détecteur pour le premier et le deuxième angle de rotation. Le troisième terme

$$\frac{1}{2nm}\left(\sum_{i=1}^{n} c_i + \sum_{j=1}^{m} \rho_j\right)$$

est constant et est calculé une seule fois pour toutes les valeurs recherchées Cij. Les opérations élémentaires mises en oeuvre par la formule conduisent à un gain de temps très important. De surcroît, les coefficients Cij sont calculés indépendamment d'une image à l'autre, en ce sens qu'aucune rétro- projection n'est effectuée entre deux images obtenues sous deux paires d'angles de rotation. L'image de synthèse est simplement le résultat d'une moyenne terme à terme des valeurs Cij des images obtenues pour les différentes paires d'angle. Cela conduit à une réduction très importante des erreurs de calcul.

L'invention s'applique au procédé de scannographie qui vient d'être décrit en ce que l'on effectue le traitement des données provenant des intensités détectées par la bande 27 du détecteur 9, irradiée ou illuminée par une nappe des rayons réfléchis par le réflecteur 15, diminuées des intensités détectées par la bande 27 du détecteur irradiée ou illuminée en l'absence du réflecteur 15.

Grâce à l'utilisation du réflecteur 15, toutes les nappes parallèles formées par les rayons réfléchis 8 sont exploitables alors que, pendant l'irradiation ou l'illumination, la source 5 est maintenue dans une position donnée par rapport au détecteur 9.

Le dispositif schématisé par la figure 1 est adapté à la mise en oeuvre du procédé de scannographie selon l'invention.

Dans un premier cas, le dispositif est conçu pour explorer une région de la tête d'une personne. L'aire de détection 23 du détecteur 9 s'étend par exemple sur 6 cm en largeur et sur 25 cm parallèlement à l'axe de rotation $\Omega$. En supposant que toute la largeur du détecteur 9 est utile, la bande du détecteur contenue dans le plan de coupe s'étend également sur 6 cm. Pour disposer de 36 images réalisant une exploration sous 360 degrés, on répète les étapes (1) à (3) du procédé pour 36 paires d'angles, décalées deux à deux de 10 degrés. La distance entre la source 5 et le détecteur 9 est ainsi égale à 68 cm. Le réflecteur 15 est un paraboloïde de révolution de paramètre $\rho$ = 1 pour que la section de sortie du paraboloïde O soit un cercle de 6 cm de diamètre lorsque la section de sortie O est disposée à 18 cm du foyer F tube à rayons X.

Dans un deuxième cas, le détecteur 9 comprend par exemple une barrette de détection 27, du type ATMEL AT71957 de 23 cm x 0,6 cm, soit une bande de détection 27 de 23 cm de largeur. La source 5 étant dans une position donnée, le détecteur 9 est déplacé verticalement par le vérin à course d'ajustement 21 sur 20 cm en deux courses d'ajustement de chacune 10 cm, à une vitesse de 8,1 cm/s pour une définition d'image de 81 $\mu$m ou à une vitesse de 2,7 cm/s pour une définition d'image de 27 $\mu$m. L'aire de détection 23 correspondant au déplacement de la barrette de détection 27 s'étend ainsi sur 23 cm en largeur et sur 20 cm parallèlement à l'axe de rotation $\Omega$. Là encore, le réflecteur 15 est un paraboloïde de révolution de paramètre $\rho$ = 1 pour que la section de sortie O du paraboloïde soit un cercle de 20 cm de diamètre lorsque la section de sortie est disposée à 50 cm du foyer F du tube à rayons X. Si le détecteur 9 comprend deux barrettes de détection 27 totalisant une largeur de 46 cm, on prévoit de déplacer horizontalement la source 5 pour réaliser deux images successives représentant la totalité de la largeur du détecteur. Pour faciliter la délimitation des deux images, on prévoit de disposer une grille de référence devant le détecteur 9.

### Revendications

**1.** Procédé d'imagerie X ou infrarouge, du type radiographie ou scannographie, dans lequel, un corps à examiner (3) étant reçu par un support (1),

- on irradie ou illumine le corps à examiner (3) à l'aide d'une source (5) émettant un faisceau (7) de rayons X ou lumineux, réfléchis en nappes parallèles par un réflecteur (15) disposé entre la source (5) et le corps à examiner (3),
- on détecte une intensité atténuée en fonction d'une traversée des rayons X ou lumineux à travers le corps à examiner (3), à l'aide d'un détecteur (9) irradié ou illuminé par le faisceau,
- on convertit les intensités détectées en données pour déterminer une atténuation par le corps à examiner des rayons X ou lumineux, à l'aide d'un convertisseur (11) analogique-numérique, et
- à l'aide d'un ordinateur (13) dûment programmé, on traite les données provenant de la conversion des intensités détectées pour aboutir à une image exprimant l'atténuation des rayons X ou lumineux par le corps examiné (3),

**caractérisé en ce que** :

- on irradie ou illumine le corps à examiner (3) une deuxième fois en l'absence du réflecteur (15), et
- on effectue le traitement des données provenant des intensités détectées par le détecteur (9) irradié ou illuminé en présence du réflecteur (15), diminuées des intensités détectées par le détecteur (9) irradié ou illuminé en l'absence du réflecteur (15).

2. Procédé d'imagerie X ou infrarouge selon la revendication 1, **caractérisé en ce que** :

- on irradie ou illumine un fantôme représentatif du corps à examiner (3) en l'absence du réflecteur (15),
- on irradie ou illumine le fantôme en présence du réflecteur (15), et
- on irradie ou illumine le corps à examiner (3) en présence du réflecteur (15).

3. Procédé d'imagerie X ou infrarouge selon la revendication 1, **caractérisé en ce que** l'on traite les données provenant de la conversion des intensités détectées pour deux positions différentes du corps à examiner (3) relativement à l'ensemble constitué par la source (5), le réflecteur (15) et le détecteur (9) pour obtenir deux images complémentaires formées à partir des rayons réfléchis (8) pour l'une et l'autre position.

4. Procédé d'imagerie X ou infrarouge du type scannographie selon la revendication 1, 2 ou 3, dans lequel :

- on fait tourner d'un angle de rotation le support (1) monté mobile autour d'un axe de rotation ($\Omega$) par rapport à la source (5) et au détecteur (9) montés sur un bâti (17) ou tourner d'un angle de rotation la source et le détecteur montés sur un bâti mobile autour d'un axe de rotation par rapport au support,
- on convertit les intensités détectées en données pour déterminer une atténuation par le corps à examiner des rayons X ou lumineux dans un plan de coupe (P) perpendiculaire à l'axe de rotation ($\Omega$), à l'aide du convertisseur (11) analogique-numérique, et
- à l'aide de l'ordinateur (13) dûment programmé, on effectue les étapes suivantes :

(1) construire un vecteur générateur colonne et un vecteur générateur ligne dont les termes sont respectivement constitués par les données ($c_i$) et ($\rho_j$) provenant de la conversion des intensités détectées dans une bande (27) du détecteur (9) pour respectivement un premier et un deuxième angle de rotation, de préférence différents entre eux de 90 degrés, du support (1) ou du bâti mobile autour de l'axe de rotation, les données étant moyennées respectivement en n et m valeurs moyennes correspondant à un quadrillage en n x m zones élémentaires du plan de coupe (P) du corps à examiner (3), perpendiculaire à l'axe de rotation ($\Omega$) et contenant la bande (27) du détecteur (9),
(2) construire une matrice initiale (n,m) avec les termes des deux vecteurs générateurs, en affectant à chaque zone élémentaire un terme de ligne et de colonne (Bij) représentant un coefficient d'atténuation et défini par la demi-somme, du terme homologue ($c_i$) du vecteur générateur colonne divisé par le nombre (m) de termes du vecteur générateur ligne et du terme homologue ($\rho_j$) du vecteur générateur ligne divisé par le nombre (n) de termes du vecteur générateur colonne,

$$B_{ij} = \frac{1}{2}\left(\frac{\rho_j}{n} + \frac{c_i}{m}\right)$$

(3) ajuster le coefficient d'atténuation en chaque zone élémentaire en utilisant la formule suivante :

$$C_{ij} = \frac{\rho_j}{n} + \frac{c_i}{m} - \frac{1}{2nm}\left(\sum_{i=1}^{n} c_i + \sum_{j=1}^{m} \rho_j\right)$$

où, dans cette formule,
Cij = la valeur recherchée du coefficient d'atténuation de la zone élémentaire (i,j) du quadrillage

Bij = la valeur estimée initialement
(n) = le nombre de lignes de la matrice initiale
(m) = le nombre de colonnes de la matrice initiale
$\rho_j$ est le j-ème terme du vecteur générateur de ligne
$c_i$ est le i-ème terme du vecteur générateur de colonne
pour aboutir à une image du plan de coupe du corps examiné sous les premier et deuxième angles de rotation, correspondant à une matrice ajustée pour laquelle les valeurs de bordure de ligne et de colonne calculées à l'aide des valeurs ajustées (Cij) sont égales, pour chaque ligne et pour chaque colonne, respectivement aux termes des vecteurs générateurs ligne et colonne,

$$\sum_{j=1}^{m} Cij = c_i$$

$$\sum_{i=1}^{n} Cij = \rho_j$$

(4) répéter les étapes (1) à (3) pour des données acquises avec différentes paires d'angles de rotation pour aboutir respectivement à différentes matrices ajustées correspondant à différentes images du plan de coupe du corps examiné sous les différentes paries d'angles de rotation, et

(5) à l'aide d'un opérateur de rotation, superposer sur une même paire d'angles toutes les matrices ajustées, et

(6) afficher à l'écran de l'ordinateur une image de synthèse du plan de coupe du corps examiné correspondant à une matrice de synthèse des coefficients d'atténuation en chaque zone élémentaire (i,j) du quadrillage, obtenus par une moyenne terme à terme de toutes les matrices ajustées et superposées, **caractérisé en ce que**,

(7) on effectue le traitement des données provenant des intensités détectées par la bande (27) du détecteur (9) irradiée ou illuminée par une nappe des rayons réfléchis (8) par le réflecteur (15), diminuées des intensités détectées par la bande (27) du détecteur irradiée ou illuminée en l'absence du réflecteur (15).

5. Dispositif d'imagerie X ou infrarouge, spécialement conçu pour la mise en oeuvre d'un procédé selon l'une des revendications 1 à 4, comprenant :

- un support (1) pour recevoir un corps à examiner (3),
- une source (5) émettant un faisceau (7) de rayons X ou lumineux, réfléchis en nappes parallèles par un réflecteur (15) disposé entre la source (5) et le corps à examiner (3),
- un détecteur (9) irradié ou illuminé par le faisceau, pour détecter une intensité atténuée en fonction d'une traversée des rayons X ou lumineux à travers le corps à examiner (3),
- un convertisseur (11) analogique-numérique pour convertir les intensités détectées en données pour déterminer une atténuation par le corps à examiner (3) des rayons X ou lumineux, et
- un ordinateur (13) dûment programmé pour traiter les données provenant de la conversion des intensités détectées pour aboutir à une image exprimant l'atténuation des rayons X ou lumineux par le corps examiné (3),

**caractérisé en ce que** :

- le réflecteur (15) est monté amovible par rapport à la source (5) pour irradier ou illuminer le corps à examiner (3) en l'absence et en présence du réflecteur,

et **en ce que** :

- l'ordinateur (13) est dûment programmé pour effectuer le traitement des données provenant des intensités détectées par le détecteur (9) irradié ou illuminé en présence du réflecteur (15), diminuées des intensités détectées par le détecteur (9) irradié ou illuminé en l'absence du réflecteur (15).

6. Dispositif d'imagerie X ou infrarouge selon la revendication 5, **caractérisé en ce que** le réflecteur comprend une succession de troncs de cône étagés (15G-15J), choisis pour minimiser à chaque étage un écart par rapport à un arc d'une parabole de référence.

7. Dispositif d'imagerie X ou infrarouge selon la revendication 5, **caractérisé en ce que** le réflecteur comprend une succession de troncs de cônes (15K-15M) revêtus intérieurement d'une épaisseur (16K-16M) de métal lourd variant suivant un arc de parabole de référence.

8. Dispositif d'imagerie X ou infrarouge selon la revendication 5, **caractérisé en ce que** le réflecteur comprend une succession de troncs de pyramide étagés de section carrée (15A-15C), hexagonale (15D-15F) ou octogonale, choisis pour minimiser à chaque étage un écart par rapport à un arc d'une parabole de référence.

9. Dispositif d'imagerie X ou infrarouge selon la revendication 7 ou 8, **caractérisé en ce que** les troncs de cônes ou les troncs de pyramide s'ouvrent en deux demi troncs montés rétractables par rapport à la source (5).

10. Programme d'ordinateur comprenant des instructions qui, lorsqu'il est chargé dans un ordinateur (13) d'un dispositif selon l'une des revendications 5 à 9, permettent à cet ordinateur (13) de traiter des données provenant de la conversion d'intensités détectées par un détecteur (9) irradié ou illuminé par un faisceau de rayons X ou lumineux, réfléchis en nappes parallèles par un réflecteur disposé entre une source à rayons X ou lumineux et un corps à examiner, pour aboutir à une image exprimant l'atténuation des rayons X ou lumineux par le corps examiné (3), **caractérisé en ce qu'**il comprend des instructions pour permettre à l'ordinateur (13) de traiter les données provenant des intensités détectées par le détecteur (9) irradié ou illuminé en présence du réflecteur (15), diminuées des intensités détectées par le détecteur (9) irradié ou illuminé en l'absence du réflecteur (15).

11. Programme d'ordinateur comprenant des instructions qui, lorsqu'il est chargé dans un ordinateur (13) à l'aide duquel un procédé selon l'une des revendications 1 à 4 est mis en oeuvre, permettent à cet ordinateur (13) de traiter des données provenant de la conversion d'intensités détectées par un détecteur (9) irradié ou illuminé par un faisceau de rayons X ou lumineux, réfléchis en nappes parallèles par un réflecteur disposé entre une source à rayons X ou lumineux et un corps à examiner, pour aboutir à une image exprimant l'atténuation des rayons X ou lumineux par le corps examiné (3), **caractérisé en ce qu'**il comprend des instructions pour permettre à l'ordinateur (13) de traiter les données provenant des intensités détectées par le détecteur (9) irradié ou illuminé en présence du réflecteur (15), diminuées des intensités détectées par le détecteur (9) irradié ou illuminé en l'absence du réflecteur (15).

**Claims**

1. X-ray or infrared imaging method, for radiography or scanning, in which a body to be examined (3) is received by a support (1) and in which,

   - the body to be examined (3) is irradiated or illuminated by a source (5) emitting a beam (7) of X-rays or light rays, reflected in parallel layers by a reflector (15) laid out between the source (5) and the body to be examined (3),
   - an intensity that is attenuated according to the passage of the X-rays or light rays through the body to be examined (3) is detected by a detector (9) irradiated or illuminated by the beam,
   - the detected intensities are converted into data enabling an attenuation of the X-rays or light rays by the body, to be determined by an analogue-to-digital converter (11), and
   - the data resulting from the conversion of the detected intensities are processed by using a programmed computer (13) in order to obtain an image representing the attenuation of the X-rays or light rays by the examined body (3),

   **characterised in that**:

   - the body to be examined (3) is irradiated or illuminated a second time without the reflector (15), and
   - the processed data result from the intensities detected by the detector (9) irradiated or illuminated with the reflector (15), decreased by the intensities detected by the detector (9) irradiated or illuminated without the reflector (15).

2. X-ray or infrared imaging method according to claim 1, **characterised in that**:

- a phantom representative of the body to be examined (3) is irradiated or illuminated without the reflector (15),
- the phantom is irradiated or illuminated with the reflector (15), and
- the body to be examined (3) is irradiated or illuminated with the reflector (15).

3. X-ray or infrared imaging method according to claim 1, **characterised in that** the processed data result from the conversion of the intensities detected for two different positions of the body (3) with respect to the unit consisting in the source (5), the reflector (15) and the detector (9), to obtain two complementary images resulting from the reflected rays (8) in one and the other position.

4. X-ray or infrared imaging method for scanning according to claim 1, 2 or 3, wherein:

- the support (1) is rotating and turned by a rotation angleabout a rotation axis ($\Omega$) with respect to the source (5) and the detector (9) mounted on a stand (17), or the source and the detector are mounted on a rotating stand and turned by a rotation angle about a rotation axis with respect to the support, and
- the detected intensities are converted into data enabling an attenuation of the X-rays or light rays by the body to be determined in a sectional plane (P) perpendicular to the rotation axis ($\Omega$), by means of an analogue-to-digital converter (11), and
- the following steps are carried out with the aid of a suitably programmed computer:

(1) to build a column generating vector and a line generating vector whose terms respectively consist in data ($c_i$) and ($\rho_j$) resulting from the conversion of intensities detected in a strip (27) of the detector (9) respectively for a first and a second rotation angle, preferably orthogonal, of the mobile support (1) or stand (17) about the rotation axis ($\Omega$), wherein said data are respectively averaged in n and m mean values corresponding to a grid of n x m elementary areas of the sectional plane (P) of the body to examine, wherein sais sectional plane is perpendicular to the rotation axis ($\Omega$) and contains the strip (27) of the detector (9),

(2) to build an initial matrix (n, m) with the terms of the two generating vectors, by assigning to each elementary area a line and column term (Bij) which represents an attenuation coefficient and is equal to the half sum of the homologous term ($c_i$) of the column generating vector divided by the number (m) of terms of the line generating vector and the homologous term ($\rho_j$) of the line generating vector divided by number (n) of terms of the column generating vector,

$$B_{ij} = \frac{1}{2}\left(\frac{\rho_j}{n} + \frac{c_i}{m}\right)$$

(3) to adjust the attenuation coefficient of each elementary area by using the following formula:

$$C_{ij} = \frac{\rho_j}{n} + \frac{c_i}{m} - \frac{1}{2nm}\left(\sum_{i=1}^{n} c_i + \sum_{j=1}^{m} \rho_j\right)$$

where, in this formula,
Cij = the desired value of the attenuation coefficient of the elementary area (i, j) of the grid
Bij = the initially estimated value
(n) = the line number of the initial matrix
(m) = the column number of the initial matrix
$\rho_j$ is j-term of the line generating vector
$c_i$ is i-term of the column generating vector
to obtain an image of the body sectional plane for the first and second angles of rotation, corresponding to an adjusted matrix for which the line and column border values calculated using the adjusted values (Cij) are respectively equal, for every line and every column, to the terms of the line and column generating vectors:

$$\sum_{j=1}^{m} Cij = c_i$$

$$\sum_{i=1}^{n} Cij = \rho_j$$

(4) to repeat steps (1) to (3) with data acquired for different pairs of rotation angles to respectively obtain different adjusted matrixes corresponding to different images of the body sectional plane for the different pairs of rotation angles,

(5) to use a rotation operator to superimpose all the adjusted matrixes on a same pair of rotation angles, and

(6) to display on the screen of the computer a synthesis image of the body sectional plane corresponding to a synthesis matrix of the attenuation coefficients of every elementary areas (i, j) of the grid, obtained by a point to point average of all the adjusted and superimposed matrixes,

**characterised in that**,

(7) the processed data result from the intensities detected by the strip (27) of the detector (9) irradiated or illuminated by a layer of rays reflected by the reflector (15), decreased by the intensities detected by the strip (27) of the detector (9) irradiated or illuminated without the reflector (15).

**5.** X-ray or infrared imaging apparatus specifically designed for the implementation of a method according to claims 1, 2, 3 or 4, comprising:

- a support (1) to receive a body to be examined (3),
- a source (5) emitting a beam (7) of X-rays or light rays, reflected by in parallel layers by a reflector (15) laid out between the source (5) and the body to be examined (3),
- a detector (9) irradiated or illuminated by the beam, to detect an intensity attenuated according to the passage of the X-rays or light rays through the body to be examined (3),
- an analogue-to-digital converter (11) to convert the detected intensities into data enabling an attenuation of the X-rays or light rays by the body to be determined, and
- a programmed computer (13) to process the data resulting from the conversion of the detected intensities in order to obtain an image representing the attenuation of the X-rays or light rays by the examined body (3),

**characterised in that**:

- the reflector (15) is removable with respect to the source (5) to irradiate or illuminate the body to be examined (3) without or with the reflector,

and **in that**:

- the computer (13) is duly programmed to process the data resulting from the intensities detected by the detector (9) irradiated or illuminated with the reflector (15), decreased by the intensities detected by the detector (9) irradiated or illuminated without the reflector (15).

**6.** X-ray or infrared imaging apparatus according to claim 5, **characterised in that** the reflector comprises a succession of staged truncated cones (15G-15J), to minimize at each stage a variation with respect to an arc of a reference parabola.

**7.** X-ray or infrared imaging apparatus according to claim 5, **characterised in that** the reflector comprises a succession of truncated cones (15K-15M) inwardly covered by a layer of heavy metal (16K-16M) that varies as an arc of a reference parabola.

**8.** X-ray or infrared imaging apparatus according to claim 5, **characterised in that** the reflector comprises a succession of staged truncated pyramids having a square (15A-15C), hexagonal (15D-15F) or octagonal section, to minimize at each stage a variation with respect to an arc of a reference parabola.

9. X-ray or infrared imaging apparatus according to claim 7 or 8, **characterised in that** the truncated cones or the truncated pyramids open into two half trunks that are retractable with respect to the source (5).

10. Computer program comprising instructions that, when loaded in a computer (13) of an apparatus according to one of claims 5 to 9, cause the computer (13) to process data resulting from the conversion of intensities detected by a detector (9) irradiated or illuminated by a beam of X-rays or light rays, reflected in parallel layers by a reflector laid out between a X-ray or light ray source and a body to be examined, to obtain an image representing the attenuation of the X-rays or light rays by the examined body (3), **characterised in that** it comprises instructions that cause the computer (13) to process the data resulting from the intensities detected by the detector (9) irradiated or illuminated with the reflector (15), decreased by the intensities detected by the detector (9) irradiated or illuminated without the reflector (15).

11. Computer program comprising instructions that, when loaded in a computer (13) with the help of which a method according to one of claims 1 to 4 is carried out, cause the computer (13) to process data resulting from the conversion of intensities detected by a detector (9) irradiated or illuminated by a beam of X-rays or light rays, reflected in parallel layers by a reflector laid out between a X-ray or light ray source and a body to be examined, to obtain an image representing the attenuation of the X-rays or light rays by the examined body (3), **characterised in that** it comprises instructions that cause the computer (13) to process the data resulting from the intensities detected by the detector (9) irradiated or illuminated with the reflector (15), decreased by the intensities detected by the detector (9) irradiated or illuminated without the reflector (15).

**Patentansprüche**

1. Röntgen -oder infrarot bildgebendes Verfahren, für Röntgen oder Scannen, wobei ein Körper (3), der zu untersuchen ist, auf einer Unterstützung (1) steht und wobei,

- der Körper (3), mittels einer Quelle (5) bestrahlt oder beleuchtet wird, die Röntgenstrahlen oder Lichtstrahlen ausstrahlt, die in parallelen Flächen von einem zwischen der Quelle (5) und dem Körper (3) stehenden Reflektor (15) reflektiert werden,
- eine beim Durchgang der Röntgenstrahlen oder Lichtstrahlen durch den Körper geschwächte Intensität, mittels eines von den Röntgenstrahlen oder Lichtstrahlen bestrahlten oder beleuchteten Detektor (9) detektiert wird,
- die detektierten Intensitäten, mittels eines Analog-Digital Konverter (11) in Daten konvertiert werden, um eine Schwächung der Röntgenstrahlen oder Lichtstrahlen beim Durchgang durch den Körper (3) zu bestimmen,
- die von der Konvertierung der detektierten Intensitäten kommenden Daten, mittels eines ordnungsgemäß programmierten Computers (13) verarbeitet werden, um zu einem Bild zu führen, das die Schwächung der Röntgenstrahlen oder Lichtstrahlen beim Durchgang durch den Körper (3) darstellt,

**dadurch gekennzeichnet, dass**:

- der Körper (3) ohne den Reflektor (15) zum zweiten Mal bestrahlt oder beleuchtet wird, und
- die verarbeitenden Daten von Intensitäten kommen, die mit dem Reflektor (15) von dem bestrahlten oder beleuchteten Detektor (9) detektiert werden und durch Intensitäten verringert sind, die ohne den Reflektor (15) mit dem bestrahlten oder beleuchteten Detektor (9) detektiert werden.

2. Röntgen -oder infrarot bildgebendes Verfahren nach Anspruch 1, **dadurch gekennzeichnet, dass**:

- ein den Körper (3) darstellendes Phantom, ohne den Reflektor (15) bestrahlt oder beleuchtet wird,
- das Phantom mit dem Reflektor (15) bestrahlt oder beleuchtet wird, und
- der Körper (3) mit dem Reflektor (15) bestrahlt oder beleuchtet wird.

3. Röntgen -oder infrarot bildgebendes Verfahren nach Anspruch 1, **dadurch gekennzeichnet, dass** die verarbeiteten Daten von der Konvertierung Intensitäten kommen, die für zwei unterschiedlichen Positionen des Körpers (3) bezüglich der Quelle (5)-Reflektor (15)- Detektor (9) Einheit detektiert werden, um zu zwei ergänzenden Bildern zu führen, die von den für die beide Positionen reflektierten Strahlen (8) kommen.

4. Röntgen oder infrarot bildgebendes Verfahren für Scannen nach Anspruch 1, 2 oder 3, wobei:

- die Quelle (5) und der Detektor (9) an einem Stand (17) angebracht sind und die Unterstützung (1) bezüglich der Quelle und des Detektors (9) in einem Rotationswinkel bezüglich einer Rotationsachse (Ω) gedreht wird, oder die Quelle (5) und der Detektor (9) an einem drehbaren Stand (17) angebracht sind und bezüglich der Unterstützung (1) in einem Rotationswinkel bezüglich einer Rotationsachse (Ω) gedreht werden, und

- die detektierten Intensität mittels eines Analog-Digital Konverter (11) in Daten konvertiert werden, um eine Schwächung den Röntgenstrahlen oder Lichtstrahlen beim Durchgang durch den Körper (3) in einer senkrecht auf der Rotationsachse (Ω) stehenden Abschnittfläche (P) zu bestimmen, und

- die folgenden Schritte mittels eines ordnungsgemäß programmierten Computers durchgeführt werden:

(1) ein Spalte erzeugender Vektor und ein Linie erzeugender Vektor zu erstellen, deren Koordinaten aus ($c_i$) und ($\rho_j$) Daten bestehen, die von der Konvertierung der in einem Streifen (27) des Detektors (9) detektierten Intensitäten kommen, beziehungsweise für einen ersten und einen zweiten Rotationswinkel, vorzugsweise orthogonal eine zum anderer, der drehbaren Unterstützung (1) oder des drehbaren Standes (17) bezüglich der Rotationsachse (Ω), wobei die Daten beziehungsweise in n und m Mittelwerten berechnet werden, die einem Rasterfeld von n x m grundlegenden Bereichen der senkrecht auf der Rotationsachse (Ω) stehenden und den Streifen (27) des Detektors (9) enthaltenden Schnittfläche (P) des Körpers entsprechen,

(2) eine initiale Matrix (n, m) mit den Koordinaten der zwei erzeugenden Vektoren so zu erstellen, dass jedem grundlegenden Bereich eine Linie und Spalte Koordinate (Bij) entspricht, die einen Schwächungskoeffizient darstellt und der halben Summe der homologen Koordinate ($c_i$) des Spalte Vektors, geteilt durch die Zahl (m) der Linie Vektor Koordinaten und der homologen Koordinate ($\rho_j$) des Linie Vektors, geteilt durch die Zahl (n) der Spalte Vektor Koordinaten, gleich ist,

$$B_{ij} = \frac{1}{2}\left(\frac{\rho_j}{n} + \frac{c_i}{m}\right)$$

(3) den Schwächungskoeffizient jedes grundlegenden Bereichs durch die Verwendung der folgenden Formel anzugleichen:

$$C_{ij} = \frac{\rho_j}{n} + \frac{c_i}{m} - \frac{1}{2nm}\left(\sum_{i=1}^{n} c_i + \sum_{j=1}^{m} \rho_j\right)$$

wo, in dieser Formel,

Cij = der gewünschte Wert des Schwächungskoeffizienten des grundlegenden Bereichs (i, j) des Rasterfeldes

Bij = der initiale abgeschätzte Wert

(n) = die Linien Zahl der initialen Matrix

(m) = die Spalten Zahl der initialen Matrix

$\rho_j$ ist die j-Koordinate des Linie erzeugenden Vektors

$c_i$ ist die i-Koordinate des Spalte erzeugenden Vektors,

um zu einem Bild der Schnittfläche (P) des Körpers für die ersten und zweiten Rotationswinkel zu führen, das einer angeglichenen Matrix entspricht, in derer die mit den angeglichenen Werten (Cij) berechneten Linie und Spalte Randwerte, beziehungsweise den Koordinaten der Linie und Spalte erzeugenden Vektoren für jede Linie und jede Spalte gleich sind:

$$\sum_{j=1}^{m} Cij = c_i$$

$$\sum_{i=1}^{n} Cij = \rho_j$$

(4) Schritte (1) bis (3) mit den Daten zu wiederholen, die für unterschiedliche Paare Rotationswinkeln ermittelt werden, um beziehungsweise zu unterschiedlichen angeglichenen Matrizen zu führen, die unterschiedlichen Bildern der Körperschnittfläche für die unterschiedlichen Paare Rotationswinkeln entsprechen,

(5) alle angeglichenen Matrizen auf einem gleichen Paar Rotationswinkeln übereinander zu legen, mittels eines Rotationsoperators, und

(6) ein Synthesebild der Körperschnittfläche auf dem Schirm des Computers anzuzeigen, das einer Synthesematrix der Schwächungskoeffizienten jedes grundlegenden Bereiches (i, j) des Rasterfeldes entspricht, die durch eine Punkt für Punkt Durchschnittberechnung aller angeglichenen und übereinandergelegten Matrizen ermittelt sind,

**dadurch gekennzeichnet dass**,

(7) die verarbeitenden Daten von Intensitäten kommen, die von dem Streife (27) des Detektors (9) detektiert werden, der von Strahlen bestrahlt oder beleuchtet wird, die von dem Reflektor (15) reflektiert werden und durch Intensitäten verringert sind, die von dem Streife (27) des Detektors (9) detektiert werden, der ohne den Reflektor (15) bestrahlt oder beleuchtet wird.

5. Röntgen -oder infrarot bildgebende Vorrichtung, besonders bestimmt für die Anwendung eines Verfahrens nach Anspruch 1, 2, 3 oder 4, mit:

   - einer Unterstützung (1), um einen Körper (3) zu unterstützen,
   - einer Quelle (5), die Röntgenstrahlen oder Lichtstrahlen ausstrahlt, die in parallelen Flächen von einem zwischen der Quelle (5) und dem Körper (3) stehenden Reflektor (15) reflektiert werden,
   - einem von den Röntgenstrahlen oder Lichtstrahlen bestrahlten oder beleuchteten Detektor (9), um eine beim Durchgang der Röntgenstrahlen oder Lichtstrahlen durch den Körper geschwächte Intensität zu detektieren,
   - einem Analog-Digital Konverter (11), das die detektierten Intensitäten in Daten konvertiert, um eine Schwächung der Röntgenstrahlen oder Lichtstrahlen beim Durchgang durch den Körper (3) zu bestimmen, und
   - einem ordnungsgemäß programmierten Computer (13), der die von der Konvertierung der detektierten Intensitäten kommenden Daten verarbeitet, um zu einem Bild zu führen, das die Schwächung der Röntgenstrahlen oder Lichtstrahlen beim Durchgang durch den Körper (3) darstellt,

   **dadurch gekennzeichnet, dass**:

   - der Reflektor (15) bezüglich der Quelle (5) demontiert ist, um den Körper (3) ohne oder mit dem Reflektor (15) zu bestrahlen oder beleuchten,

   und dass:

   - der Computer (13) ordnungsgemäß programmiert ist, um Daten zu verarbeiten, die von Intensitäten kommen, die von dem mit dem Reflektor (15) bestrahlten oder beleuchteten Detektor (9) detektiert werden, und durch Intensitäten verringert sind, die von dem ohne den Reflektor (15) bestrahlten oder beleuchteten Detektor (9) detektiert werden.

6. Röntgen -oder infrarot bildgebende Vorrichtung nach Anspruch 5, **dadurch gekennzeichnet, dass** der Reflektor eine Reihenfolge mehrerer abgestuften Kegelstümpfen (15G-15J) aufweist, die auf jeder Stufe eine Veränderung bezüglich eines Kreisbogens einer Vergleichsparabel minimal halten.

7. Röntgen -oder infrarot bildgebende Vorrichtung nach Anspruch 5, **dadurch gekennzeichnet, dass** der Reflektor eine Reihenfolge mehreren Kegelstümpfen (15K-15M) aufweist, die innerlich von einer Schicht Schwermetall (16K-16M) abgedeckt sind, die sich als einen Kreisbogen einer Vergleichsparabel ändert.

8. Röntgen -oder infrarot bildgebende Vorrichtung nach Anspruch 5, **dadurch gekennzeichnet, dass** der Reflektor eine Reihenfolge mehreren abgestuften Pyramidestümpfen (15G-15J) aufweist, die eine viereckige, oder sechseckige, oder achteckige Grundfläche (15A-15C) aufweisen und auf jeder Stufe eine Veränderung bezüglich eines Kreisbogens einer Vergleichsparabel minimal halten.

9. Röntgen -oder infrarot bildgebende Vorrichtung nach Anspruch 7 oder 8, **dadurch gekennzeichnet, dass** sich die abgestuften Kegelstümpfen oder Pyramidestümpfen in zwei halbe Strümpfen öffnen, die bezüglich der Quelle (5) einziehbar sind.

10. Computerprogramm mit Befehlen die, wenn das Programm in einem Computer (13) einer Vorrichtung nach einem von Ansprüchen 5 bis 9 eingelesen wird, es diesem Computer (13) ermöglichen, Daten zu verarbeiten, die von der Konvertierung detektierter Intensitäten kommen, die von einem Detektor (9) detektiert werden, der von Röntgenstrahlen oder Lichtstrahlen bestrahlt oder beleuchtet wird, die in parallelen Flächen von einem zwischen einer Quelle und einem Körper stehenden Reflektor reflektiert werden, um zu einem Bild zu führen, das die Schwächung der Röntgenstrahlen oder Lichtstrahlen beim Durchgang durch den Körper (3) darstellt, **dadurch gekennzeichnet, dass** das Programm Befehle aufweist, die es dem Computer (13) ermöglichen, die Daten zu verarbeiten, die von Intensitäten kommen, die von dem mit dem Reflektor (15) bestrahlten oder beleuchteten Detektor (9) detektiert werden und durch Intensitäten verringert sind, die von dem ohne den Reflektor (15) bestrahlten oder beleuchteten Detektor (9) detektiert werden.

11. Computerprogramm mit Befehlen die, wenn das Programm in einem für ein Verfahren nach einem von Ansprüchen 1 bis 4 eingesetzten Computer (13) eingelesen wird, es diesem Computer (13) ermöglichen, Daten zu verarbeiten, die von der Konvertierung detektierter Intensitäten kommen, die von einem Detektor (9) detektiert werden, der von Röntgenstrahlen oder Lichtstrahlen bestrahlt oder beleuchtet wird, die in parallelen Flächen von einem zwischen einer Quelle und einem Körper stehenden Reflektor reflektiert werden, um zu einem Bild zu führen, das die Schwächung der Röntgenstrahlen oder Lichtstrahlen beim Durchgang durch den Körper (3) darstellt, **dadurch gekennzeichnet, dass** das Programm Befehle aufweist, die es dem Computer (13) ermöglichen, die Daten zu verarbeiten, die von Intensitäten kommen, die von dem mit dem Reflektor (15) bestrahlten oder beleuchteten Detektor (9) detektiert werden und durch Intensitäten verringert sind, die von dem ohne den Reflektor (15) bestrahlten oder beleuchteten Detektor (9) detektiert werden.

Fig. 1

Fig. 2

| x | y | A | B | C = B - A |
|---|---|---|---|---|
| 2,000 | 2,000 | 26,565 | 45,000 | 18,435 |
| 3,000 | 2,449 | 22,208 | 39,231 | 17,024 |
| 4,000 | 2,828 | 19,471 | 35,264 | 15,793 |
| 5,000 | 3,162 | 17,548 | 32,311 | 14,763 |
| 6,000 | 3,464 | 16,102 | 30,000 | 13,898 |
| 7,000 | 3,742 | 14,963 | 28,125 | 13,162 |
| 8,000 | 4,000 | 14,036 | 26,565 | 12,529 |
| 9,000 | 4,243 | 13,263 | 25,239 | 11,977 |
| 10,000 | 4,472 | 12,604 | 24,095 | 11,490 |
| 11,000 | 4,690 | 12,035 | 23,093 | 11,058 |
| 12,000 | 4,899 | 11,537 | 22,208 | 10,671 |
| 13,000 | 5,099 | 11,096 | 21,417 | 10,321 |
| 14,000 | 5,292 | 10,702 | 20,705 | 10,003 |
| 15,000 | 5,477 | 10,347 | 20,060 | 9,713 |
| 16,000 | 5,657 | 10,025 | 19,471 | 9,446 |
| 17,000 | 5,831 | 9,731 | 18,932 | 9,200 |
| 18,000 | 6,000 | 9,462 | 18,435 | 8,973 |
| 19,000 | 6,164 | 9,214 | 17,975 | 8,761 |
| 20,000 | 6,325 | 8,985 | 17,548 | 8,564 |
| 21,000 | 6,481 | 8,772 | 17,151 | 8,379 |
| 22,000 | 6,633 | 8,573 | 16,779 | 8,206 |
| 23,000 | 6,782 | 8,387 | 16,430 | 8,043 |
| 24,000 | 6,928 | 8,213 | 16,102 | 7,889 |
| 25,000 | 7,071 | 8,049 | 15,793 | 7,744 |
| 26,000 | 7,211 | 7,895 | 15,501 | 7,606 |
| 27,000 | 7,348 | 7,749 | 15,225 | 7,476 |
| 28,000 | 7,483 | 7,611 | 14,963 | 7,352 |
| 29,000 | 7,616 | 7,480 | 14,714 | 7,234 |
| 30,000 | 7,746 | 7,356 | 14,477 | 7,121 |
| 31,000 | 7,874 | 7,238 | 14,252 | 7,014 |
| 32,000 | 8,000 | 7,125 | 14,036 | 6,911 |
| 33,000 | 8,124 | 7,017 | 13,830 | 6,813 |
| 34,000 | 8,246 | 6,914 | 13,633 | 6,719 |
| 35,000 | 8,367 | 6,816 | 13,444 | 6,628 |
| 36,000 | 8,485 | 6,721 | 13,263 | 6,541 |
| 37,000 | 8,602 | 6,631 | 13,088 | 6,458 |
| 38,000 | 8,718 | 6,544 | 12,921 | 6,377 |
| 39,000 | 8,832 | 6,460 | 12,760 | 6,300 |
| 40,000 | 8,944 | 6,379 | 12,604 | 6,225 |
| 41,000 | 9,055 | 6,302 | 12,455 | 6,153 |
| 42,000 | 9,165 | 6,227 | 12,310 | 6,083 |
| 43,000 | 9,274 | 6,155 | 12,170 | 6,016 |
| 44,000 | 9,381 | 6,085 | 12,035 | 5,951 |
| 45,000 | 9,487 | 6,017 | 11,905 | 5,887 |
| 46,000 | 9,592 | 5,952 | 11,778 | 5,826 |
| 47,000 | 9,695 | 5,889 | 11,656 | 5,767 |
| 48,000 | 9,798 | 5,828 | 11,537 | 5,709 |
| 49,000 | 9,899 | 5,768 | 11,422 | 5,654 |
| 50,000 | 10,000 | 5,711 | 11,310 | 5,599 |
| 51,000 | 10,100 | 5,655 | 11,201 | 5,547 |
| 52,000 | 10,198 | 5,600 | 11,096 | 5,495 |
| 53,000 | 10,296 | 5,548 | 10,993 | 5,446 |
| 54,000 | 10,392 | 5,496 | 10,893 | 5,397 |
| 55,000 | 10,488 | 5,446 | 10,796 | 5,350 |
| 56,000 | 10,583 | 5,398 | 10,702 | 5,304 |
| 57,000 | 10,677 | 5,351 | 10,610 | 5,259 |
| 58,000 | 10,770 | 5,305 | 10,520 | 5,215 |
| 59,000 | 10,863 | 5,260 | 10,432 | 5,172 |
| 60,000 | 10,954 | 5,216 | 10,347 | 5,131 |
| 61,000 | 11,045 | 5,173 | 10,263 | 5,090 |
| 62,000 | 11,136 | 5,132 | 10,182 | 5,051 |
| 63,000 | 11,225 | 5,091 | 10,103 | 5,012 |
| 64,000 | 11,314 | 5,051 | 10,025 | 4,974 |
| 65,000 | 11,402 | 5,012 | 9,949 | 4,937 |
| 66,000 | 11,489 | 4,974 | 9,875 | 4,901 |
| 67,000 | 11,576 | 4,937 | 9,802 | 4,865 |
| 68,000 | 11,662 | 4,901 | 9,731 | 4,830 |
| 69,000 | 11,747 | 4,866 | 9,662 | 4,796 |
| 70,000 | 11,832 | 4,831 | 9,594 | 4,763 |
| 71,000 | 11,916 | 4,797 | 9,527 | 4,731 |
| 72,000 | 12,000 | 4,764 | 9,462 | 4,699 |
| 73,000 | 12,083 | 4,731 | 9,398 | 4,667 |
| 74,000 | 12,166 | 4,699 | 9,336 | 4,637 |
| 75,000 | 12,247 | 4,668 | 9,274 | 4,607 |
| 76,000 | 12,329 | 4,637 | 9,214 | 4,577 |
| 77,000 | 12,410 | 4,607 | 9,155 | 4,548 |
| 78,000 | 12,490 | 4,578 | 9,097 | 4,520 |
| 79,000 | 12,570 | 4,549 | 9,041 | 4,492 |
| 80,000 | 12,649 | 4,520 | 8,985 | 4,465 |
| 81,000 | 12,728 | 4,492 | 8,930 | 4,438 |
| 82,000 | 12,806 | 4,465 | 8,876 | 4,411 |
| 83,000 | 12,884 | 4,438 | 8,824 | 4,385 |
| 84,000 | 12,961 | 4,412 | 8,772 | 4,360 |
| 85,000 | 13,038 | 4,386 | 8,721 | 4,335 |
| 86,000 | 13,115 | 4,360 | 8,671 | 4,310 |
| 87,000 | 13,191 | 4,335 | 8,621 | 4,286 |
| 88,000 | 13,266 | 4,311 | 8,573 | 4,262 |
| 89,000 | 13,342 | 4,286 | 8,525 | 4,239 |
| 90,000 | 13,416 | 4,263 | 8,479 | 4,216 |
| 91,000 | 13,491 | 4,239 | 8,433 | 4,193 |
| 92,000 | 13,565 | 4,216 | 8,387 | 4,171 |
| 93,000 | 13,638 | 4,194 | 8,343 | 4,149 |
| 94,000 | 13,711 | 4,171 | 8,299 | 4,128 |
| 95,000 | 13,784 | 4,149 | 8,256 | 4,106 |
| 96,000 | 13,856 | 4,128 | 8,213 | 4,085 |
| 97,000 | 13,928 | 4,107 | 8,171 | 4,065 |
| 98,000 | 14,000 | 4,086 | 8,130 | 4,044 |
| 99,000 | 14,071 | 4,065 | 8,089 | 4,024 |
| 100,000 | 14,142 | 4,045 | 8,049 | 4,005 |

Fig. 3

22

Fig. 4

Fig. 5

Fig. 6

| x | y | y' | y'' | y''(x) / y''(50) | a | b | Δ | σ | ε |
|---|---|---|---|---|---|---|---|---|---|
| **8,00** | 4,00 | 0,25 | -0,02 | **15,62** | | | **0,00** | | |
| 9,00 | 4,24 | 0,24 | -0,01 | 13,09 | | | -0,02 | | |
| 10,00 | 4,47 | 0,22 | -0,01 | 11,18 | 0,220 | 2,242 | -0,03 | 5,41E-03 | 1,19E-03 |
| 11,00 | 4,69 | 0,21 | -0,01 | 9,69 | | | -0,03 | | |
| 12,00 | 4,90 | 0,20 | -0,01 | 8,51 | | | -0,02 | | |
| **13,00** | 5,10 | 0,20 | -0,01 | **7,54** | | | **0,00** | | |
| 14,00 | 5,29 | 0,19 | -0,01 | 6,75 | | | -0,02 | | |
| 15,00 | 5,48 | 0,18 | -0,01 | 6,09 | | | -0,03 | | |
| 16,00 | 5,66 | 0,18 | -0,01 | 5,52 | | | -0,03 | | |
| 17,00 | 5,83 | 0,17 | -0,01 | 5,04 | 0,175 | 2,823 | -0,03 | 3,25E-16 | 5,69E-17 |
| 18,00 | 6,00 | 0,17 | 0,00 | 4,63 | | | -0,03 | | |
| 19,00 | 6,16 | 0,16 | 0,00 | 4,27 | | | -0,01 | | |
| **20,00** | 6,32 | 0,16 | 0,00 | **3,95** | | | **0,00** | | |
| 21,00 | 6,48 | 0,15 | 0,00 | 3,67 | | | -0,02 | | |
| 22,00 | 6,63 | 0,15 | 0,00 | 3,43 | | | -0,03 | | |
| 23,00 | 6,78 | 0,15 | 0,00 | 3,21 | | | -0,04 | | |
| 24,00 | 6,93 | 0,14 | 0,00 | 3,01 | | | -0,04 | | |
| 25,00 | 7,07 | 0,14 | 0,00 | 2,83 | | | -0,04 | | |
| 26,00 | 7,21 | 0,14 | 0,00 | 2,67 | 0,141 | 3,507 | -0,04 | 1,01E-14 | 1,42E-15 |
| 27,00 | 7,35 | 0,14 | 0,00 | 2,52 | | | -0,04 | | |
| 28,00 | 7,48 | 0,13 | 0,00 | 2,39 | | | -0,03 | | |
| 29,00 | 7,62 | 0,13 | 0,00 | 2,26 | | | -0,02 | | |
| 30,00 | 7,75 | 0,13 | 0,00 | 2,15 | | | -0,01 | | |
| **31,00** | 7,87 | 0,13 | 0,00 | **2,05** | | | **0,00** | | |
| 32,00 | 8,00 | 0,13 | 0,00 | 1,95 | | | -0,01 | | |
| 33,00 | 8,12 | 0,12 | 0,00 | 1,87 | | | -0,03 | | |
| 34,00 | 8,25 | 0,12 | 0,00 | 1,78 | | | -0,04 | | |
| 35,00 | 8,37 | 0,12 | 0,00 | 1,71 | | | -0,05 | | |
| 36,00 | 8,49 | 0,12 | 0,00 | 1,64 | | | -0,05 | | |
| 37,00 | 8,60 | 0,12 | 0,00 | 1,57 | | | -0,06 | | |
| 38,00 | 8,72 | 0,11 | 0,00 | 1,51 | | | -0,06 | | |
| 39,00 | 8,83 | 0,11 | 0,00 | 1,45 | | | -0,06 | | |
| 40,00 | 8,94 | 0,11 | 0,00 | 1,40 | | | -0,06 | | |
| 41,00 | 9,06 | 0,11 | 0,00 | 1,35 | 0,112 | 4,405 | -0,06 | 4,64E-03 | 5,19E-04 |
| 42,00 | 9,17 | 0,11 | 0,00 | 1,30 | | | -0,06 | | |
| 43,00 | 9,27 | 0,11 | 0,00 | 1,25 | | | -0,06 | | |
| 44,00 | 9,38 | 0,11 | 0,00 | 1,21 | | | -0,05 | | |
| 45,00 | 9,49 | 0,11 | 0,00 | 1,17 | | | -0,05 | | |
| 46,00 | 9,59 | 0,10 | 0,00 | 1,13 | | | -0,04 | | |
| 47,00 | 9,70 | 0,10 | 0,00 | 1,10 | | | -0,03 | | |
| 48,00 | 9,80 | 0,10 | 0,00 | 1,06 | | | -0,02 | | |
| 49,00 | 9,90 | 0,10 | 0,00 | 1,03 | | | -0,01 | | |
| **50,00** | 10,00 | 0,10 | 0,00 | **1,00** | | | **0,00** | | |

Fig. 7

Fig. 8

15M    16M

15L    16L

16K

15K

Fig. 9

24

A

18L

20L

16L

15L

22

B

30

28

26

Fig. 10

Fig. 11

Fig. 12

Fig. 13

## RÉFÉRENCES CITÉES DANS LA DESCRIPTION

**Documents brevets cités dans la description**

- WO 2006003311 A
- DE 10139384 A
- WO 2004100790 A
- WO 2006107130 A
- FR 2888374
- WO 2007006560 A
- FR 2871911
- WO 2006003312 A